# EUROPEAN PATENT APPLICATION

(11) **EP 4 170 679 A1**
(43) Date of publication of application: **26.04.2023**
(21) Application number: 20946214.2
(22) Date of filing: 23.07.2020
(51) Int. Cl.: G16H 50/20, G16H 50/50, G16H 50/70, G16H 30/20, G06N 3/08, G06N 20/00

(54) **METHOD AND SYSTEM FOR PROVIDING CUSTOMIZED DIAGNOSIS SYSTEM**

(71) Applicant: Deep Bio Inc., Seoul 08394 (KR)
(72) Inventor: KIM, Sun Woo, Seongnam-si, Gyeonggi-do 13599 (KR)
(74) Representative: Flügel Preissner Schober Seidel
(86) International application number: PCT/KR2020/009719
(87) International publication number: WO 2022/019354

(57) **Abstract**

Disclosed are a method and a system for providing a customized diagnosis system. A method for providing a diagnosis system, which is to achieve the technical task, comprises the steps wherein: a reference diagnosis system transmits, to a first diagnostic subject system, M (M is an integer of 2 or larger) pieces of unannotated learning data for customization; the reference diagnosis system receives the M pieces of learning data for customization, which are annotated by a first diagnostic subject side, from the first diagnostic subject system; and the received annotated M pieces of learning data for customization are reflected in the reference diagnosis system, wherein the M pieces of learning data for customization, which have been annotated by the first diagnostic subject side, are reflected in a neural network for the reference diagnosis system under a condition that a predetermined price is to be paid.

## Description

### Technical Field

The present disclosure relates to a method and system for diagnosing a disease through learning. More specifically, the present disclosure relates to a method capable of implementing a customized diagnosis system reflecting diagnostic propensity of a specific diagnoser using a diagnosis system trained based on supervised learning through a neural network and a system therefor.

### Background Art

One of the main tasks performed by the pathology or pathology department is to perform diagnosis to determine the condition or symptom of a specific disease by reading a patient's biometric image. This diagnosis is a method that relies on the experience and knowledge of skilled medical personnel for a long time.

Recently, due to the development of machine learning, attempts to automate tasks such as recognizing or classifying images by computer systems have been actively made. In particular, attempts have been made to automate diagnosis performed by skilled medical personnel using a neural network (e.g., a deep learning method using a convolutional neural network (CNN)), which is a type of machine learning.

In particular, diagnosis through deep learning using a neural network (e.g., CNN) does not simply automate the experience and knowledge of conventionally skilled medical personnel, but in that it finds the characteristic elements through self-learning and derives the desired answer, in some cases, the characteristics of disease factors that skilled medical personnel were not aware of may be found in images.

In general, diagnosis of a disease through a neural network using biometric data (e.g., biometric image) is performed by annotating biometric data with the state of a specific disease (e.g., whether or not a cancer has been manifested) by a skilled medical personnel, and a neural network is trained by using such a plurality of annotated data as training data. In other words, learning through the annotated training data by performing annotation on the training data for learning is mainly used, and this learning method is called supervised learning.

However, in supervised learning, the performance or propensity of the trained system is dominantly influenced by the judgment propensity or tendency of the annotator who performed the annotation. In other words, diagnosis results of the trained diagnosis system depend on the annotation tendency or propensity of the annotator.

However, it is a reality that there is a considerable amount of biometric data that is difficult to be clearly classified in the biometric data to be learned, to the extent that different annotations are performed depending on the annotator when performing actual annotation. In other words, even if skilled medical personnel perform annotation, opinions may differ in classifying whether or not a disease has been manifested or the degree of progression of a disease according to biometric data.

Nevertheless, in the case of a disease diagnosis system trained based on supervised learning, there is a weakness in outputting a judgment dependent on the annotator's tendency as a diagnosis result.

In addition, in order to construct a diagnosis system using a neural network, a plurality of training data and an annotation action by a skilled medical person to annotate the plurality of training data are required, which requires a lot of time and money.

Therefore, there are practical difficulties for small medical institutions to construct a diagnosis system using such a trained neural network. In addition, even if a neural network trained by another institution is purchased or leased and used, as described above, since neural networks are dependent on annotations, it is difficult to construct a diagnosis system that reflects one's own diagnosis propensity.

Therefore, technical ideas that may solve these problems are required.

### [Prior Art Document]

- Patent Document : Korean Patent Application No. 10-2017-0057399 "Disease diagnosis system through modularized reinforcement learning"

### Disclosure of the Invention

### Technical Goals

A technical object to be achieved by the present disclosure is to provide a method and system for providing a diagnosis system having a customized propensity to a diagnoser (diagnostic institution).

In addition, it is to provide a method and system for performing diagnosis using the customized diagnosis system.

In addition, it is to provide a method and system capable of supplementing the weaknesses of the supervised learning, by being provided with a plurality of supervised learning-based diagnosis systems trained by different annotation subjects, and deriving a final diagnosis result, i.e., a consensus diagnosis result between the diagnosis systems based on diagnosis results performed by the plurality of diagnosis systems for predetermined biometric data.

In addition, it is to provide a method and system capable of improving the performance of individual diagnosis systems by allowing the individual diagnosis systems to be re-trained using the diagnosis result through the consensus of these diagnosis systems. Technical Solutions

A method for providing a customized diagnosis system for achieving the above technical objects includes transmitting, by a reference diagnosis system, M (M is an integer of 2 or more) pieces of unannotated training data for customization to a first diagnoser system, receiving, by the reference diagnosis system, the M pieces of training data for customization annotated by a first diagnoser side from the first diagnoser system, and reflecting the received M pieces of annotated training data for customization in the reference diagnosis system, wherein the M pieces of training data for customization annotated by the first diagnoser side are reflected in a neural network for the reference diagnosis system under a condition that a predetermined price is paid.

The method for providing a customized diagnosis system may include receiving diagnosis data from the first diagnoser system, generating a diagnosis result, and transmitting the diagnosis result to the first diagnoser system, by the reference diagnosis system in which the M pieces of annotated training data for customization are reflected.

The reference diagnosis system may set the price to be larger as the M is larger or according to a ratio of the M to the number of training data learned by the reference diagnosis system.

The reference diagnosis system may be a system that includes an individual diagnosis system set including a plurality of individual diagnosis systems and performs diagnosis through consensus based on individual diagnosis results output by the plurality of individual diagnosis systems.

The reflecting of the received M pieces of annotated training data for customization in the reference diagnosis system may include re-training at least some of the plurality of individual diagnosis systems using the M pieces of annotated training data for customization.

The M pieces of training data for customization may include data in which at least two of the individual diagnosis systems included in the individual diagnosis system set output different diagnosis results.

A method for providing a customized diagnosis system for solving the above technical problems includes transmitting, by a reference diagnosis system, M (M is an integer of 2 or more) pieces of unannotated training data for customization to a first diagnoser system, the reference diagnosis system including an individual diagnosis system set including a plurality of individual diagnosis systems, receiving, by the reference diagnosis system, the M pieces of training data for customization annotated by a first diagnoser side from the first diagnoser system, reflecting the received M pieces of annotated training data for customization in any one first individual diagnosis system included in the reference diagnosis system set, receiving a diagnosis request including diagnosis data to be diagnosed by the reference diagnosis system from a predetermined diagnosis request system, and determining, according to the received diagnosis request, a type of diagnosis result corresponding to the diagnosis request as a first individual diagnosis result of the first individual diagnosis system, and transmitting a diagnosis result including the first individual diagnosis result output by the first individual diagnosis system receiving input data to the diagnosis request system.

The above method may be implemented by a computer program stored in a non-transitory computer readable recording medium installed in a data processing device.

A system for solving the above technical problems includes a processor, a storage device in which a program executed by the processor is stored, wherein the storage device includes at least one neural network, and the program is configured to transmit M (M is an integer of 2 or more) pieces of unannotated training data for customization to a first diagnoser system, receive the M pieces of training data for customization annotated by a first diagnoser side from the first diagnoser system, and reflect the received M pieces of annotated training data for customization in a reference diagnosis system, and wherein the M pieces of training data for customization annotated by the first diagnoser side are reflected in the neural network under a condition that a predetermined price is paid.

A system for solving the above technical problem includes a processor, a storage device in which a program executed by the processor is stored, wherein the storage device includes a neural network corresponding to a plurality of individual diagnosis systems, and wherein the program is configured to transmit M (M is an integer of 2 or more) pieces of unannotated training data for customization to a first diagnoser system, and when receiving the M pieces of training data for customization annotated by a first diagnoser side from the first diagnoser system, reflect the received M pieces of annotated training data for customization to a first neural network corresponding to a first individual diagnosis system among the plurality of individual diagnosis systems, and when receiving a diagnosis request including diagnosis data to be diagnosed from a predetermined diagnosis request system, determine, according to the received diagnosis request, a type of diagnosis result corresponding to the diagnosis request as a first individual diagnosis result of a first individual diagnosis system, and transmit a diagnosis result including the first individual diagnosis result output by the first individual diagnosis system receiving input data to the diagnosis request system.

### Advantageous Effects

According to the technical idea of the present disclosure, there is an effect of easily constructing a customized diagnosis system reflecting the propensity of a specific diagnoser using a reference diagnosis system.

In addition, by providing such a customized diagnosis system, there is an effect of generating new revenue.

In addition, when diagnosing a disease through a supervised learning-based diagnosis system trained using training data annotated by an annotation subject, there is an effect of supplementing the annotator dependency of the diagnosis result.

In addition, according to the technical idea of the present disclosure, individual diagnosis systems may be re-trained using diagnosis results through consensus, and in this case, there is as effect that the performance of individual diagnosis systems may also be improved.

### Brief Description of Drawings

In order to more fully understand the drawings cited in the detailed description of the present disclosure, a brief description of each drawing is provided.
FIG. 1 is a diagram for explaining a schematic concept for implementing a method for providing a customized diagnosis system in accordance with an embodiment of the present disclosure.
FIG. 2 is a diagram for exemplarily explaining an implementation example of a reference diagnosis system in accordance with an embodiment of the present disclosure.
FIG. 3 is a diagram for explaining a concept for customizing a reference diagnosis system in accordance with an embodiment of the present disclosure.
FIG. 4 is a diagram showing a schematic system configuration when a reference diagnosis system performs a supervised learning-based consensus diagnosis method in accordance with an embodiment of the present disclosure.
FIG. 5 is a diagram for explaining a concept of utilizing a customized diagnosis system in accordance with an embodiment of the present disclosure.
FIG. 6 is a diagram for explaining a schematic configuration of each of diagnosis systems in accordance with an embodiment of the present disclosure.
FIG. 7 is a diagram for explaining a concept in which a reference diagnosis system derives a consensus based on individual diagnosis results in accordance with an embodiment of the present disclosure.
FIG. 8 is a diagram for explaining a concept of split annotation of training data in accordance with an embodiment of the present disclosure.
FIG. 9 is a diagram for explaining a concept of re-training individual diagnosis systems using a consensus diagnosis result in accordance with an embodiment of the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. The terms are used only for the purpose of distinguishing one component from another.

The terms used in the present application are used only to describe a particular embodiment and are not intended to limit the present disclosure. Singular expressions include plural expressions unless the context clearly means otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail centering on embodiments of the present disclosure. Like reference numerals in each figure indicate like members.

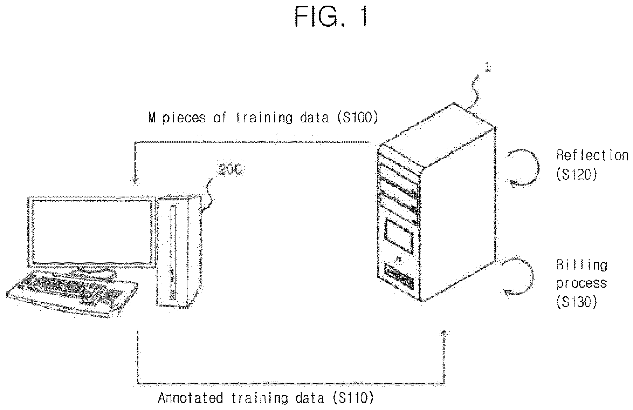

FIG. 1 is a diagram for explaining a schematic concept for implementing a method for providing a customized diagnosis system in accordance with an embodiment of the present disclosure.

Referring to FIG. 1, a reference diagnosis system 1 may be implemented to implement a method for providing a customized diagnosis system according to an embodiment of the present disclosure.

The reference diagnosis system 1 may provide a customized diagnosis system according to the technical idea of the present disclosure.

According to one example, the reference diagnosis system 1 may be changed into a customized diagnosis system by itself. Alternatively, the reference diagnosis system 1 may include a plurality of neural networks, and some of the plurality of neural networks may be implemented into the customized diagnosis system.

Thus, the reference diagnosis system 1 may include one or a plurality of neural networks.

The reference diagnosis system 1 may implement the technical idea of the present disclosure while communicating with a predetermined diagnoser (defined as meaning including a diagnosis institution)-side system (e.g., a first diagnoser system 200) wanting to construct a customized diagnosis system.

The first diagnoser system 200 refers to a system used by the first diagnoser, and may not necessarily be specified as any one physical device.

The reference diagnosis system 1 may be a diagnosis system trained using predetermined training data. In this way, when using the reference diagnosis system 1 trained to some extent in advance, as will be described later, the first diagnoser system 200 may perform annotation only on a relatively small amount of training data.

Depending on the embodiment, the reference diagnosis system 1 may perform training at once when additional annotated training data is received from the first diagnoser system 200 in a state in which only training data is prepared.

According to an example, the reference diagnosis system 1 may be a system trained in advance through a plurality of training data. The reference diagnosis system 1 may be a system trained to output a judgment on a disease, i.e., diagnosis result, when training data, for example, a biometric image is input. Training data used for training of the reference diagnosis system 1 may be data annotated by someone other than the first diagnoser.

In addition, the reference diagnosis system 1 may retain some unannotated training data.

Then, among the unannotated training data, M (M is an integer of 2 or more) pieces of training data may be transmitted to the first diagnoser system 200 (S 100).

The M pieces of training data may be training data used to reflect the diagnosis propensity of the first diagnoser system 200, i.e., the first diagnoser, to the reference diagnosis system 1. Therefore, M pieces of training data transmitted to the first diagnoser system 200 may be defined as training data for customization.

The number of training data for customization, i.e., M, may be predetermined. In addition, M may be determined in association with the number of training data used for training of the reference diagnosis system 1. In other words, it is because if the number of training data for customization that is too small than the number of training data used for training of the reference diagnosis system 1 is used, the diagnosis propensity of the first diagnoser may not be well expressed in the reference diagnosis system 1.

For example, if the reference diagnosis system 1 is a diagnosis system trained with 10000 pieces of training data, the M may be determined to be about 200 pieces. The determination of the number of M may reflect the propensity of the first diagnoser through experiments, and may be determined to a number that is not too large.

When the M pieces of training data for customization are transmitted to the first diagnoser system 200, the M pieces of training data for customization may be annotated by the first diagnoser. Further, the annotated training data for customization may be transmitted from the first diagnoser system 200 to the reference diagnosis system 1 (S 110).

According to an embodiment, the M pieces of training data for customization may not be randomly selected, but may be data selectively selected through a predetermined process. For example, training data having ambiguous characteristics in which diagnosis results may slightly differ for each diagnoser may be selected in advance as training data for customization. For example, the training data may be a biometric image, and depending on the biometric image, a specific diagnoser may determine that a disease has been manifested, and another diagnoser may determine that a disease has not been manifested. Alternatively, when the diagnosis system is trained to determine the extent of disease progression, a specific diagnoser may determine that the disease is in the first state and another diagnoser may determine that the disease is in the second state for the same biometric image. As such, in fact, from a pathological point of view, there may be cases in which a specific biometric image is so ambiguous that whether or not a disease has been manifested or the progress of a disease may be judged differently depending on a diagnoser.

When data including features having ambiguity is selected and used as training data for customization, even if a smaller number of customized training data is used, there is an effect that the propensity of a specific diagnoser may be better reflected. This is because, in the case of training data with features clear enough to produce the same diagnosis result, there may not be much room for the diagnosis propensity to be reflected, and when such data is used as training data for customization, it may not significantly affect the pre-trained reference diagnosis system 1.

According to an example, as will be described later, the reference diagnosis system 1 may be implemented to derive a final diagnosis result by consensus of a plurality of individual diagnosis systems, and in this case, input data from which at least two of the plurality of individual diagnosis systems output different diagnosis results may be selected as training data for customization. In other words, when the reference diagnosis system 1 is implemented as a consensus diagnosis system, it may include a plurality of individual diagnosis systems, and in this case, because each individual diagnosis system may perform the function of a diagnoser, naturally, there is an effect of being able to select data including features having ambiguity by using diagnosis results of individual diagnosis systems.

Then, the reference diagnosis system 1 may reflect the annotated training data for customization to the reference diagnosis system 1 (S120).

That the annotated training data for customization is reflected in the reference diagnosis system 1 may mean that the annotated training data for customization performs a series of data processing so that the annotated training data for customization affects the diagnosis of the reference diagnosis system 1.

For example, the reference diagnosis system 1 may reflect the training data for customization to the reference diagnosis system 1 by performing re-training or transfer learning using the received training data for customization.

As such, when the annotated training data for customization by the first diagnoser is reflected in the reference diagnosis system 1, the reference diagnosis system 1 may function as a diagnosis system in which the diagnostic propensity of the first diagnoser is reflected, i.e., customized by the first diagnoser.

Meanwhile, in return for implementing the diagnosis system customized for the first diagnoser, the subject side of the reference diagnosis system 1 may perform billing (S130). For the billing, the reference diagnosis system 1 may perform communication with the first diagnosis system 200 to perform a predetermined billing process. An average expert in the technical field of the present disclosure may easily infer that the billing process exists in a variety of ways. In addition, the billing may proceed through a separate process according to a predetermined contract rather than the billing process automated by the reference diagnosis system 1.

In addition, an average expert in the technical field of the present disclosure may easily infer that the billing may be performed before or after the training data for customization is reflected in the reference diagnosis system 1.

Meanwhile, the billing amount may be selected to be depending on the quality or number of training data for customization.

For example, when the training data for customization is randomly selected from a plurality of training data, a cheaper billing amount may be set, and when the training data including features having ambiguity is selected as described above, a higher billing amount may be set.

Alternatively, the billing amount may be selected differently according to the ratio of the number of training data for customization to the number of training data other than the training data for customization in the diagnosis system (e.g., the reference diagnosis system 1 or individual diagnosis systems as described below) to which the training data for customization is to be reflected. In general, the higher the above ratio, the more it may be customized dependently on the propensity of the first diagnoser, so a higher amount may be selected as the billing amount. Alternatively, a higher billing amount may be simply set as the number of training data for customization increases.

As such, by constructing a customized diagnosis system for the first diagnoser reflecting the diagnosis propensity of the first diagnose, there is an effect that individual diagnosis institutions need not to secure a large number of training data to construct a diagnosis system, and need not to perform annotation to the obtained training data individually, and need not to utilize these training data, and an individual diagnosis institution may be constructed without professional knowledge to construct a diagnosis system.

In addition, from the point of view of the provider of the reference diagnosis system 1, there is an effect that additional revenue may be generated by easily constructing a diagnosis system reflecting the propensity of each individual diagnoser.

In FIG. 1, although the reference diagnosis system 1 is illustrated as any one physical device, according to embodiments, the reference diagnosis system 1 may be a system in which a plurality of physical devices are organically combined.

In addition, the reference diagnosis system 1 may be provided with a plurality of individual neural networks, and each neural network may be a neural network trained to individually receive input data and output diagnosis results. Of course, depending on the embodiment, the reference diagnosis system 1 may include only one neural network, and in this case, training data for customization may be reflected in the one neural network.

Depending on the embodiment of the reference diagnosis system 1, the embodiment in which the training data for customization is reflected in the reference diagnosis system 1 may vary.

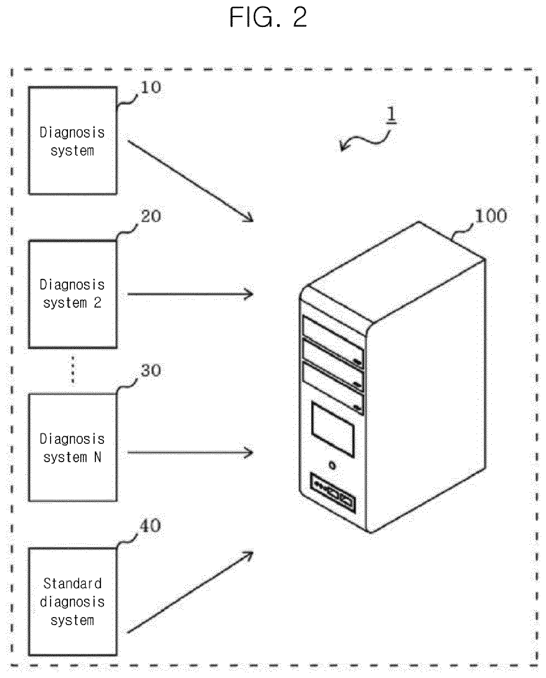

An example of this will be described with reference to FIG. 2.

FIG. 2 is a diagram for exemplarily explaining an implementation example of a reference diagnosis system in accordance with an embodiment of the present disclosure.

Referring to FIG. 2, the reference diagnosis system 1 may include a plurality of individual diagnosis systems (e.g., 10 to 30) including a plurality of neural networks, respectively. Depending on embodiments, a standard diagnosis system 40 may be further included. In addition, the reference diagnosis system 1 may include a predetermined consensus diagnosis system 100.

Each of the individual diagnosis systems (e.g., diagnosis system 1 to diagnosis system N, 10 to 30) may be a system trained to output individual diagnosis results (e.g., individual diagnosis result 1 to individual diagnosis result N). Then, as will be described later, the consensus diagnosis system 100 may be a system implemented to output a consensus diagnosis result based on these individual diagnosis results (e.g., individual diagnosis result 1 to individual diagnosis result N).

When predetermined biometric data (e.g., biometric image) is input, each of the diagnosis systems (e.g., diagnosis system 1 to diagnosis system N, 10 to 30) trained by based on supervised learning may be a system that classifies the biometric data into a predetermined classification (e.g., classification according to manifestation of disease or extent of disease progression) through a trained neural network and outputs the result as a diagnosis result.

The diagnosis systems (e.g., diagnosis system 1 to diagnosis system N, 10 to 30) may respectively receive the same biometric data and output diagnosis results, i.e., the individual diagnosis results (e.g., individual diagnosis result 1 to individual diagnosis result N) through respective trained neural networks.

In this case, a diagnosis result of each of the diagnosis systems (e.g., diagnosis system 1 to diagnosis system N, 10 to 30) may be dependent on annotation according to an annotator. In other words, each of the diagnosis systems (e.g., diagnosis system 1 to diagnosis system N, 10 to 30) may output individual diagnosis results to have dependence on the annotated training data.

In order to compensate for such weakness, each of the diagnosis systems (e.g., diagnosis system 1 to diagnosis system N, 10 to 30) according to the technical idea of the present disclosure may be a system trained with a training data set annotated by different annotators. Depending on embodiments, each of the diagnosis systems (e.g., diagnosis system 1 to diagnosis system N, 10 to 30) may be diagnosis systems trained using the same training data annotated identically. Further, each of the diagnosis systems (e.g., diagnosis system 1 to diagnosis system N, 10 to 30) may be customized by different diagnosers later.

For example, diagnosis system 1 10 may be a system trained with a first training data set annotated by a first annotator, diagnosis system 2 20 may be a system trained with a second training data set annotated by a second annotator, and diagnosis system N 30 may be a system trained with an Nth training data set annotated by an Nth annotator.

In this case, each of the diagnosis systems (e.g., diagnosis system 1 to diagnosis system N, 10 to 30) may have a characteristic that depends on the judgment propensity or tendency of the annotator of the training data used for learning.

Then, although it is desirable that the diagnosis systems (e.g., diagnosis system 1 to diagnosis system N, 10 to 30) receiving the same diagnosis data (e.g., biometric image) output the same diagnosis results ideally, as described above, the judgment propensity of the annotator may affect the diagnosis result, so that in some cases, different diagnosis results may be output. In other words, the diagnosis systems (e.g., diagnosis system 1 to diagnosis system N, 10 to 30) may output different diagnosis results for the same diagnosis data according to the propensity of the annotator.

However, such weakness may be supplemented through consensus diagnosis using individual diagnosis results of a plurality of diagnosis systems (e.g., diagnosis system 1 to diagnosis system N, 10 to 30) according to the technical idea of the present disclosure.

For example, the reference diagnosis system 1 may output a consensus diagnosis result based on individual diagnosis results output by each diagnosis system (e.g., diagnosis system 1 to diagnosis system N, 10 to 30). The consensus diagnosis result may mean a final diagnosis result generated based on all or part of the individual diagnosis results.

Depending on the embodiment, the standard diagnosis system 1 may further include a standard diagnosis system 40 to be described later. In addition, the consensus diagnosis result may be determined based further on the diagnosis result of the standard diagnosis system 40, i.e., the standard individual diagnosis result.

The standard diagnosis system 40 may refer to a diagnosis system trained with gold standard training data. Gold standard training data may refer to training data in which standard or ideal annotations have been performed. Such gold standard training data may not be simply annotated by one subject, but may refer to data on which annotations agreed upon by a plurality of subjects are performed or on which annotations are performed based on criteria agreed upon by a plurality of subjects. In general, it may be desirable to perform training using such gold standard training data, but in practice, generating gold standard training data may be expensive, and rather than constructing a single diagnosis system trained using gold standard training data, constructing a consensus diagnosis result by having a plurality of diagnosis systems trained by individual subjects may rather demonstrate higher diagnostic performance. This is because while in the case of gold standard training data, when there are different annotation opinions on annotations for specific biometric data, only the consensus annotations are reflected in training while the minority opinions are ignored, as in the technical idea of the present disclosure, when individually trained diagnosis systems (e.g., diagnosis system 1 to diagnosis system N, 10 to 30) are constructed through annotations by individual subjects, minority opinions are reflected in training, at least in individual diagnosis systems.

Moreover, when the standard diagnosis system 40 is included among the plurality of diagnosis systems for consensus diagnosis, higher diagnostic performance may be shown.

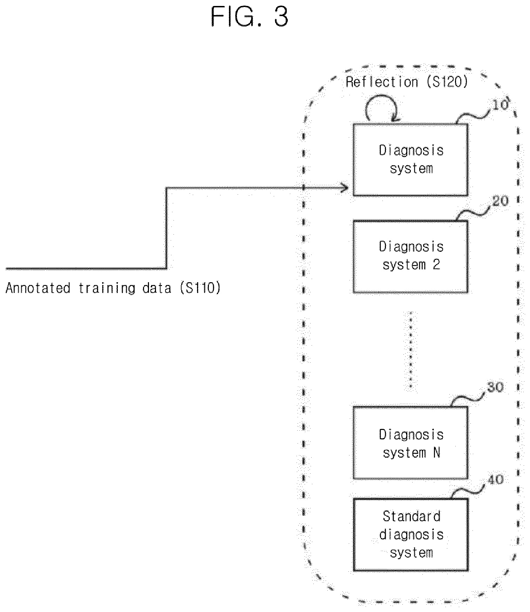

FIG. 3 is a diagram for explaining a concept for customizing a reference diagnosis system in accordance with an embodiment of the present disclosure.

Referring to FIG. 3, as described above, when a plurality of diagnosis systems (e.g., 10 to 40) are included in the reference diagnosis system 1, reference diagnosis system 1 may, after receiving M pieces of annotated training data for customization from the first diagnoser system 200 (S110), reflect the annotated training data for customization limitedly to any one diagnosis system (e.g., diagnosis systems 1, 10) among the diagnosis systems (e.g., diagnosis system 1 to diagnosis system N, 10 to 30) (S120). Of course, re-training or transfer learning may be used to reflect the annotated training data for customization.

In other words, when the reference diagnosis system 1 is provided with a plurality of diagnosis systems (e.g., diagnosis system 1 to diagnosis system N, 10 to 30), any one of the plurality of diagnosis systems (e.g., diagnosis system 1 to diagnosis system N, 10 to 30) may be customized for the first diagnoser.

Then, the customized diagnosis system 1 10 is customized for the first diagnoser, and other diagnosis systems (e.g., 20 to 40) may remain without reflecting the diagnosis propensity of the first diagnoser. In this case, when predetermined data, i.e., diagnosis data to be diagnosed is received from the first diagnoser system 200, the reference diagnosis system 1 may have an effect of providing not only diagnosis results customized for the first diagnoser, i.e., individual diagnosis results output by diagnosis system 1 10 but also diagnosis results of other diagnosis systems or consensus diagnosis results.

According to another embodiment, the annotated training data for customization received by the reference diagnosis system 1 from the first diagnoser system 200 may be reflected in all diagnosis systems (e.g., diagnosis system 1 to diagnosis system N, 10 to 30). Of course, in this case, all diagnosis systems (e.g., diagnosis system 1 to diagnosis system N, 10 to 30) may be changed to output diagnosis results reflecting the diagnosis propensity of the first diagnoser.

Of course, even at this time, the standard diagnosis system 40 may not reflect the annotated training data for customization received from the first diagnoser system 200.

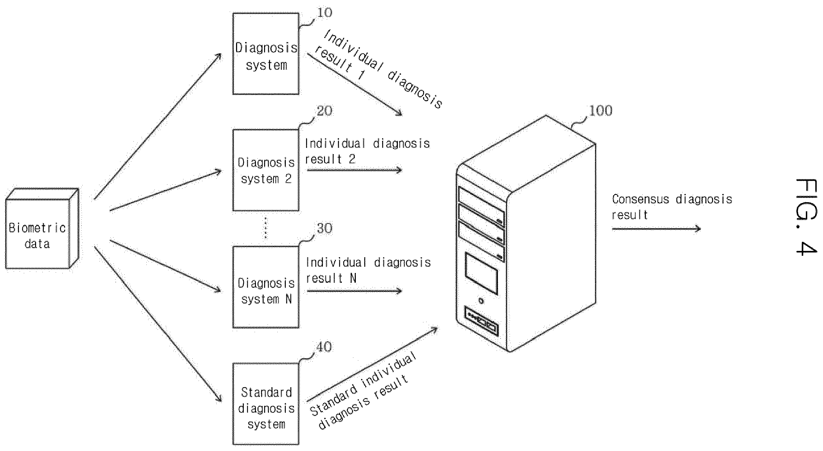

FIG. 4 is a diagram showing a schematic system configuration when a reference diagnosis system performs a supervised learning-based consensus diagnosis method in accordance with an embodiment of the present disclosure.

Referring to FIG. 4, as described above, the reference diagnosis system 1 may include a plurality of diagnosis systems (e.g., diagnosis system 1 to diagnosis system N, 10 to 30) and a consensus diagnosis system 100. In addition, a standard diagnosis system 30 may be further included.

In addition, in order to output a consensus diagnosis result, when the individual diagnosis results have different diagnosis results, the consensus diagnosis system 100 may simply derive a consensus diagnosis result according to a majority vote. Alternatively, the consensus diagnosis system 100 may assign a weight to each diagnosis system (e.g., diagnosis system 1 to diagnosis system N, 10 to 30) and output a consensus diagnosis result obtained by applying the assigned weight to a corresponding individual diagnosis result.

Weights may be assigned according to the diagnostic history of each of the diagnosis systems (e.g., diagnosis system 1 to diagnosis system N, 10 to 30). For example, a weighting process may be driven by the consensus diagnosis system 100 such that a weight is reduced by a predetermined criterion for a diagnosis system that outputs a diagnosis result different from the consensus diagnosis result. In addition, the weighting process may simply consider whether or not it is different from the consensus diagnosis result, but may further consider the number of diagnosis results by type among all individual diagnosis results.

For example, when 10 diagnosis systems are implemented to implement consensus diagnosis, among the 10 diagnosis systems, only one specific diagnosis system may determine that the disease has not been manifested for specific biometric data, and all other diagnosis systems may determine that the disease has been manifested. In this case, the weight of the specific diagnostis system may be reduced by a predetermined first reference value (e.g., 0.1).

However, 4 out of 10 diagnosis systems may determine that the disease has not been manifested for the specific biometric data, and all 6 diagnosis systems may determine that the disease has been manifested. In this case, even if the consensus diagnosis result is the manifestation of a disease, the weights of the four diagnosis systems may be reduced by a predetermined second reference value (e.g., 0.05) smaller than the first reference value (e.g., 0.1).

In other words, when the consensus diagnosis result is assumed to be the correct answer, the weight of the diagnosis system may be adjusted simply according to the number of outputting incorrect answers, but even when a specific diagnosis system outputs an incorrect answer, the degree of adjustment of the weight may be changed in consideration of the number of diagnosis systems outputting an incorrect answer, or in some cases, the weight may not be adjusted because it is determined that the answer is not incorrect.

In addition, an average expert in the technical field of the present disclosure may easily infer that various embodiments may be possible for methods for deriving consensus diagnosis results and methods for assigning weights to each of the diagnosis systems (e.g., diagnosis system 1 to diagnosis system N, 10 to 30).

Depending on the embodiment, the consensus diagnosis system 100 may further receive a standard individual diagnosis result from the standard diagnosis system 40 to be described later. Further, a consensus diagnosis result may be output based on the received standard individual diagnosis result.

In addition, the consensus diagnosis system 100 may assign a higher weight to the standard diagnosis system 40 than each of the individual diagnosis systems (e.g., diagnosis system 1 to diagnosis system N, 10 to 30), and reflect the weight assigned to each diagnosis system (e.g., diagnosis system 1 to diagnosis system N, 10 to 30) to derive a consensus result.

Meanwhile, although the case in which the consensus diagnosis system 100 is implemented as a separate physical device from the diagnosis systems (e.g., diagnosis system 1 to diagnosis system N, 10 to 30) or the standard diagnosis system 40 is shown in FIG. 4, an average expert in the technical field of the present disclosure may easily infer that the consensus diagnosis system 100 may be implemented in the physically same device as at least one of the diagnosis systems (e.g., diagnosis system 1 to diagnosis system N, 10 to 30) or the standard diagnosis system 40.

The consensus diagnosis system 100 may be implemented with various data processing systems (e.g., computers, servers, smart phones, or dedicated devices) as long as they may perform the functions defined in this specification.

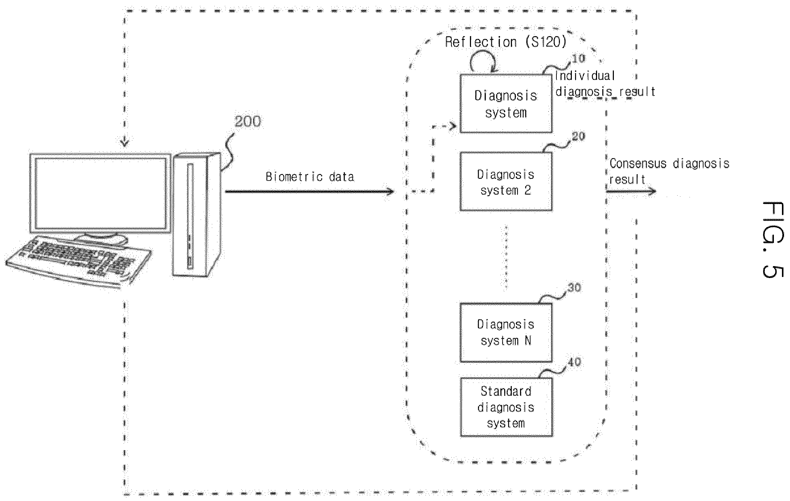

FIG. 5 is a diagram for explaining a concept of utilizing a customized diagnosis system in accordance with an embodiment of the present disclosure.

Referring to FIG. 5, the reference diagnosis system 1 may be a system in which M pieces of annotated training data for customization received from the first diagnoser system 200 are reflected. And according to an example, as described above, the reference diagnosis system 1 includes a plurality of diagnosis systems (e.g., diagnosis system 1 to diagnosis system N, 10 to 30) and/or a standard diagnosis system 40, and may be a system implemented to perform consensus diagnosis according to the individual diagnosis results thereof.

In this case, the annotated training data for customization received from the first diagnoser system 200 may be reflected only in one (e.g., diagnosis system 1 10) of a plurality of diagnosis systems (e.g., diagnosis system 1 to diagnosis system N, 10 to 30).

Thereafter, the reference diagnosis system 1 may receive diagnosis data from the diagnosis request system. The diagnosis request system may be a system intended to transmit diagnosis data (e.g., biometric data such as biometric images) to the reference diagnosis system 1 and to receive diagnosis results corresponding to the diagnosis data from the reference diagnosis system 1, i.e., to request a diagnosis to the reference diagnosis system 1. The diagnosis request system may be the first diagnoser system 200 or a system of another diagnostic institution.

The reference diagnosis system 1 may receive a diagnosis request including the diagnosis data and determine a type of diagnosis result based on the diagnosis request. The type of diagnosis result may be, for example, of which diagnosis result of the diagnosis system is being requested. For example, according to a diagnosis request, only an individual diagnosis result of a specific individual diagnosis system may be transmitted, or a consensus diagnosis result may be transmitted together.

For example, the reference diagnosis system 1 may determine whether the diagnosis request is a request for a diagnosis result through a diagnosis system customized to the first diagnoser. The diagnosis request through the diagnosis system customized for the first diagnoser may be a diagnosis request performed by the first diagnoser, but is not limited thereto, and other diagnosers may also trust the diagnosis propensity of the first diagnoser, and in this case, other diagnosers may also want a diagnosis result customized for the first diagnoser.

For example, the reference diagnosis system 1 may specify the diagnosis result type by using the properties (e.g., IP, MAC address, etc.) of the system that outputs the diagnosis request. Alternatively, when a service page for using the reference diagnosis system 1 is provided, the type of diagnosis result to be transmitted corresponding to the diagnosis request may be determined through log-in information or the like. Alternatively, information about the diagnosis result type (e.g., identification information of the first diagnoser, identification information of the diagnosis system 1, etc.) may be explicitly included in the diagnosis request.

For example, when a diagnosis request including predetermined diagnosis data is received from the first diagnoser system 200, the reference diagnosis system 1 may specify a type of diagnosis result corresponding to the diagnosis request.

Then, the reference diagnosis system 1 may transmit only the individual diagnosis result of the diagnosis system 1 10 customized to the first diagnoser to the first diagnoser system 200. Depending on the embodiment, the diagnosis of the first diagnoser may be helped by transmitting the consensus diagnosis result together with the individual diagnosis result of the diagnosis system 110. In particular, when the individual diagnosis result of the diagnosis system 1 10 differs from the consensus diagnosis result, there is an effect of notifying the first diagnoser that a more careful diagnosis is necessary.

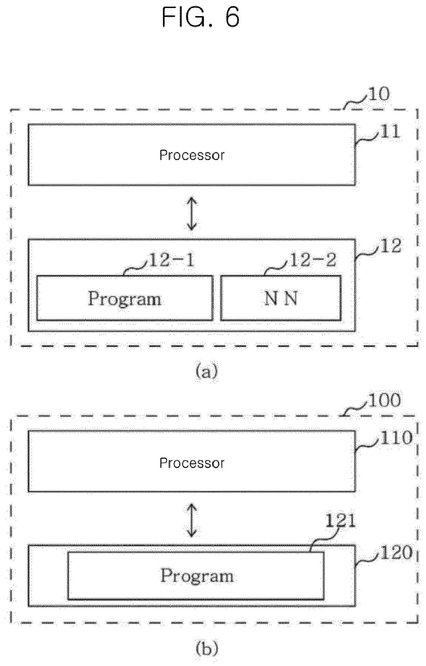

FIG. 6 is a diagram for explaining a schematic configuration of each of diagnosis systems in accordance with an embodiment of the present disclosure.

First, referring to FIG. 6A, the reference diagnosis system 1 may include only one neural network, i.e., a neural network for the reference diagnosis system, and as described above, a neural network for an individual diagnosis system corresponding to each of the plurality of diagnosis systems (e.g., diagnosis system 1 to diagnosis system N, 10 to 30) may be included. In other words, there may be a plurality of neural networks for the reference diagnosis system that are neural networks included in the reference diagnosis system 1.

The individual diagnosis systems (e.g., diagnosis systems (e.g., diagnosis system 1 to diagnosis system N, 10 to 30)) may include a configuration as shown in FIG. 6A. Since the standard diagnosis system 40 may also be implemented with the same configuration as the individual diagnosis systems (e.g., diagnosis system 1 to diagnosis system N, 10 to 30), only the individual diagnosis systems (e.g., diagnosis system 1 to diagnosis system N, 10 to 30) will be described in this specification. In addition, since individual diagnosis systems (e.g., diagnosis system 1 to diagnosis system N, 10 to 30) may also include the same or similar configuration, only one configuration of the diagnosis systems (e.g., diagnosis system 1 to diagnosis system N, 10 to 30) will be described in this specification.

The first diagnosis system (diagnosis system 1) 10 may include a processor 11 and a storage device 12. The first diagnosis system 10 refers to a data processing device having an arithmetic capability for implementing the technical idea of the present disclosure, and an average expert in the art of the present disclosure may easily infer that it may be implemented in any device capable of performing a specific service, such as a personal computer, mobile terminal, etc., as well as a data processing device that is generally accessible by a client via a network.

The processor 11 may refer to an arithmetic device capable of driving the program 12-1 for implementing the technical idea of the present disclosure, and the processor 11 may perform diagnosis using the program 12-1 and a neural network 12-2 defined by the technical idea of the present disclosure. The neural network may be a convolutional neural network, and upon receiving biometric data (e.g., an image), a diagnosis result may be output through the trained neural network.

The program 12-1 may refer to software defined to train the neural network 12-2 through supervised learning or perform diagnosis using the trained neural network 12-2.

The storage device 12 may refer to a data storage means capable of storing the program 12-1 and the neural network 12-2, and may be implemented as a plurality of storage means according to embodiments. In addition, the storage device 12 may include not only the main memory included in the first diagnosis system 10, but also a temporary storage device or memory that may be included in the processor 11.

Hereinafter, that the diagnosis system (e.g., 10) performs a predetermined function may mean that the processor (e.g., 11) provided in the diagnosis system (e.g., 10) performs a predetermined function using the program (e.g., 12-1).

In the present specification, that the first diagnosis system 10 performs diagnosis may refer to a series of processes of receiving biometric data and outputting output data defined in the present specification, i.e., a diagnosis result.

The first diagnosis system 10 may input biometric data for each predetermined unit. The unit may be, for example, a pixel unit, a patch, or a slide unit.

The diagnosis result of the first diagnosis system 10 may be simply whether or not the disease has been manifested or a value corresponding thereto (e.g., probability, etc.) depending on the type of disease, or when a disease has been manifested, it may be state information indicating the degree of the state of the disease.

For example, as will be described later, when the technical idea of the present disclosure is used for diagnosis of prostate cancer, a Gleason pattern or a Gleason score, which is an index indicating the progress of prostate cancer, may be included in the state information. For example, the Gleason pattern has a value of 2 to 5, and the higher the number, the more severe the degree of prostate cancer manifestation is. Therefore, the state information may include information corresponding to a probability that the biological tissue corresponding to the unit to be diagnosed corresponds to a specific value (e.g., 3, 4, or 5) of the Gleason pattern or information corresponding to a probability of corresponding to normal (i.e., when the disease has not been manifested).

In any case, the first diagnosis system 10 may output a diagnosis result through the trained neural network 12-2.

Meanwhile, the consensus diagnosis system 100 according to the technical idea of the present disclosure may include the configuration shown in FIG. 2B.

The consensus diagnosis system 100 may also include a storage device 120 in which the processor 110 and the program 121 are stored.

The consensus diagnosis system 100 may be connected to individual diagnosis systems (e.g., diagnosis system 1 to diagnosis system N, 10 to 30) and/or the standard diagnosis system 40 through a wired/wireless network to transmit/receive information necessary for implementing the technical idea of the present disclosure. Depending on the embodiment, the consensus diagnosis system 100 may be installed and implemented in the individual diagnosis systems (e.g., diagnosis system 1 to diagnosis system N, 10 to 30) or the standard diagnosis system 40. In this case, the consensus diagnosis system 100 may share hardware of the individual diagnosis systems (e.g., diagnosis system 1 to diagnosis system N, 10 to 30) or the standard diagnosis system 40.

The program 121 stored in the storage device 120 of the consensus diagnosis system 100 may receive and confirm diagnosis results (e.g., individual diagnosis results 1 to N and/or standard individual diagnosis results) from individual diagnosis systems (e.g., diagnosis system 1 to diagnosis system N, 10 to 30) and/or the standard diagnosis system 40, respectively.

While the consensus diagnosis system 100 may receive individual diagnosis results from only the diagnosis systems (e.g., diagnosis system 1 to diagnosis system N, 10 to 30) each trained with a training data set annotated by different annotators, as described above, a standard individual diagnosis result may be further received from the standard diagnosis system 40. As described above, each of the diagnosis systems (e.g., diagnosis system 1 to diagnosis system N, 10 to 30) may be systems trained with the same training data, and each diagnosis system may be prepared to be customized for a specific diagnoser as described above.

In any case, the consensus diagnosis system 100 may generate and output a consensus diagnosis result based on the received diagnosis results.

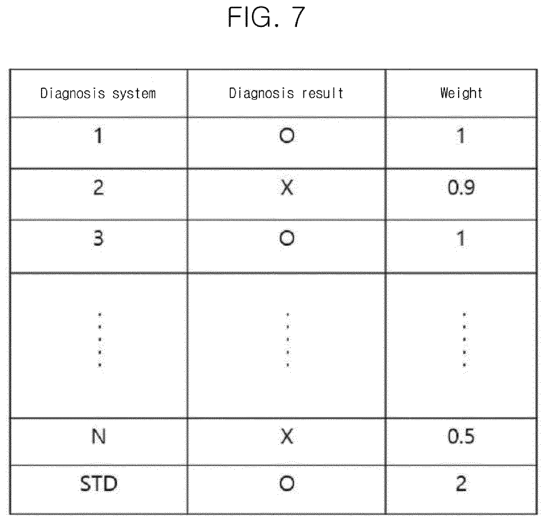

An example of generating a consensus diagnosis result by the consensus diagnosis system 100 will be described with reference to FIG. 7.

FIG. 7 is a diagram for explaining a concept of deriving a consensus based on individual diagnosis results in accordance with an embodiment of the present disclosure.

Referring to FIG. 7, diagnosis systems (e.g., diagnosis system 1 to diagnosis system N, 10 to 30) may be implemented to implement the technical idea of the present disclosure, and the consensus diagnosis system 100 included in the reference diagnosis system 1 may receive diagnosis results for the same biometric data from respective diagnosis systems (e.g., diagnosis system 1 to diagnosis system N, 10 to 30). In addition, FIG. 7 illustrates a case where the consensus diagnosis system 100 further receives a standard individual diagnosis result from the standard diagnosis system 40 (STD).

Although FIG. 7 exemplifies a case in which the diagnosis result indicates whether or not a specific disease has been manifested, as described above, various types of information may be output as diagnosis results according to the implementation example of the neural network 12-2.

According to an example, the consensus diagnosis system 100 may simply specify a type having a greater number of diagnosis result types (e.g., O where the disease has been manifested, X where the disease has not been manifested) as a consensus diagnosis result.

Depending on the embodiment, the consensus diagnosis system 100 may assign a weight to each diagnosis system (e.g., diagnosis system 1 to diagnosis system N, 10 to 30, standard diagnosis system 40).

Such a weight may be adjusted based on the number of times an individual diagnosis result different from the consensus diagnosis result is output while deriving a consensus diagnosis result for a plurality of biometric data. For example, diagnosis system 2 may have a weight of 0.9, and diagnosis system 1 may have a weight of 1, which may mean that there was at least one case where diagnosis system 2 output an individual diagnosis result different from the consensus diagnosis result.

The consensus diagnosis system 100 may generate a consensus diagnosis result based on a diagnosis result in which a weight is reflected as a weight factor. For example, if a specific diagnosis system outputs a diagnosis result as X and the weight of the specific diagnosis system is a (e.g., 0.9), in the process of deriving the consensus diagnosis result, the diagnosis result of the specific diagnosis system may be determined as a (0.9) X. In this way, more types of diagnosis results (e.g., O or X) may be determined as the consensus diagnosis result by reflecting the weight.

The consensus diagnosis system 100 may adjust the weight by lowering a predetermined value (e.g., 0.1) for each diagnosis system (e.g., diagnosis system 1 to diagnosis system N, 10 to 30 and/or standard diagnosis system 40), whenever an individual diagnosis result different from the consensus diagnosis result is simply output.

Alternatively, as described above, even when a specific diagnosis system (e.g., diagnosis system 2) output an individual diagnosis result (e.g., X) different from the consensus diagnosis result (e.g., O), the weight may be adjusted by further considering the number or proportion (the number of diagnosis systems which output the same type of diagnosis results as the specific diagnosis system (e.g., diagnosis system 2) compared to the total number of diagnosis systems) of diagnosis systems (e.g., diagnosis system 1 to diagnosis system N, 10 to 30 and/or standard diagnosis system 40) which output the other individual diagnosis results (e.g., X). For example, when only 1 out of 10 diagnosis systems output different diagnosis results, the weight of the corresponding system may be adjusted by a first value (e.g., 0.1), and when different diagnosis results are output by more than a certain number (e.g., 3) or a certain proportion (e.g., 30%) of 10 diagnosis systems, the weight of each system may be adjusted only by the second value (e.g., 0.03). Alternatively, in the case of a certain number or a certain proportion, the weight of the corresponding system may not be adjusted at all even if a diagnosis result different from the consensus diagnosis result is output.

In other words, since the latter case may be interpreted as a different diagnosis result according to actual judgment, a more reliable diagnosis result may be derived by adaptively adjusting the weight corresponding to the reliability of the corresponding diagnosis system.

In addition, when the diagnosis result of the standard diagnosis system 40 is further used, the consensus diagnosis system 100 may assign a higher weight to the standard diagnosis system 40 than other diagnosis systems (e.g., diagnosis system 1 to diagnosis system N, 10 to 30).

Meanwhile, in the case of the consensus diagnosis method according to the technical idea of the present disclosure, a diagnosis result may be usefully derived even when diagnosis systems (e.g., diagnosis system 1 to diagnosis system N, 10 to 30 and/or standard diagnosis system 40) trained with different training data sets are used. As will be described later, this may be more useful when the consensus diagnosis system 100 performs re-training (or transfer learning) on a diagnosis system that outputs a diagnosis result different from the consensus diagnosis result.

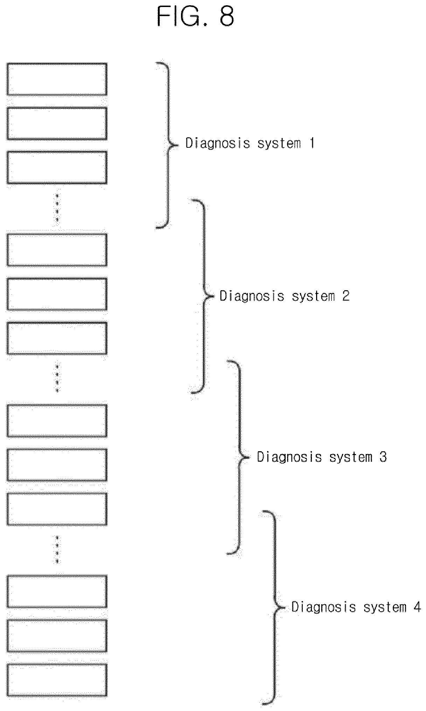

FIG. 8 is a diagram for explaining a concept of split annotation of training data in accordance with an embodiment of the present disclosure.

Referring to FIG. 8, when the diagnosis method according to the technical idea of the present disclosure is applied, as described above, it may be useful not only when each diagnosis system (e.g., diagnosis system 1 to diagnosis system N, 10 to 30 and/or standard diagnosis system 40) is trained using training data differently annotated for each annotator, but also when training is performed using different training data for each diagnosis system (e.g., diagnosis system 1 to diagnosis system N, 10 to 30 and/or standard diagnosis system 40).

For example, as shown in FIG. 8, the performance of the trained engine, i.e., the diagnosis system may vary depending on the annotator's propensity to annotate training data used for learning, but even when the same annotator performs annotation, it is also affected by which training data is used.

Therefore, as shown in FIG. 8, diagnosis system 1 may be a system trained with training data set 1, diagnosis system 2 may be a system trained with training data set 2, diagnosis system 3 may be a system trained with training data set 3, and diagnosis system 4 may be a system trained with training data set 4.

Each training data set may or may not have overlapping training data, and the annotator may be the same or different for each training data set.

In any case, there may be differences in the diagnosis results of each trained diagnosis system for each training data set, and to this end, conventionally, when selecting a training data set, considerable efforts and costs had to be spent to determine a wide range of training data sets including all of the various cases.

However, according to the technical idea of the present disclosure, if learning is performed with a training data set that is sufficient to a certain extent to train each of the diagnosis systems, even when diagnosis systems (e.g., diagnosis system 1 to diagnosis system N, 10 to 30 and/or standard diagnosis system 40) are trained with different training data sets, more accurate diagnosis results may be derived through the consensus diagnosis result.

In other words, there is an effect that the bias of the training data set itself may be significantly eliminated in the process of deriving the consensus diagnosis result.

Furthermore, for a diagnosis system that output a diagnosis result different from the consensus diagnosis result for specific biometric data, re-training may be performed by annotating the specific biometric data with a consensus diagnosis result, and in this case, the bias by the annotator and/or the bias by the training data may be eliminated over time, so that the performance of individual diagnosis systems (e.g., diagnosis system 1 to diagnosis system N, 10 to 30 and/or standard diagnosis system 40) may ultimately be improved.

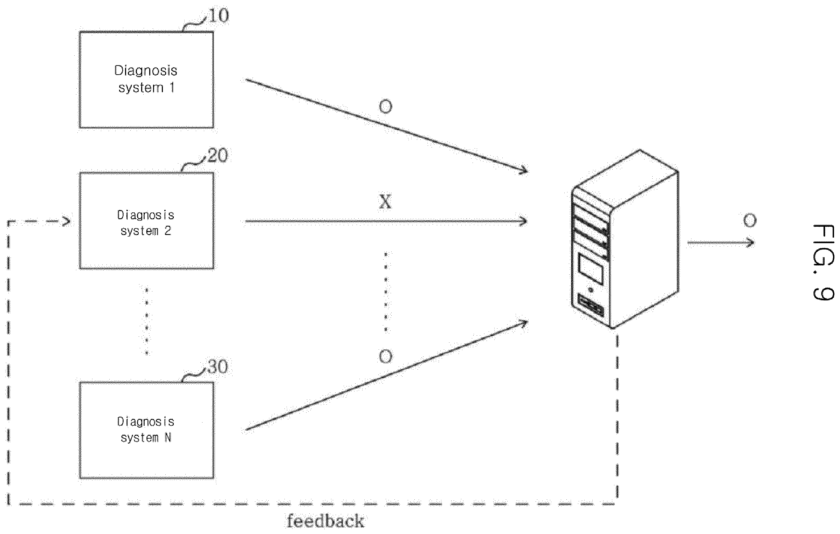

An example of this will be described with reference to FIG. 9.

FIG. 9 is a diagram for explaining a concept of re-training individual diagnosis systems using a consensus diagnosis result in accordance with an embodiment of the present disclosure.

Referring to FIG. 9, for example, specific biometric data may be input to each diagnosis system (e.g., diagnosis system 1 to diagnosis system N, 10 to 30 and/or standard diagnosis system 40), and each diagnosis system (e.g., diagnosis system 1 to diagnosis system N, 10 to 30 and/or standard diagnosis system 40) may output an individual diagnosis result to the consensus diagnosis system 100. At this time, the consensus diagnosis system 100 may have output O as the consensus diagnosis result, and the diagnosis system 2 20 may have output X as the individual diagnosis result.

In this case, the consensus diagnosis system 100 may store information about a diagnosis system (e.g., diagnosis system 2) that output an individual diagnosis result different from the consensus diagnosis result, the specific biometric data at this time, and consensus diagnosis result (e.g., O).

In addition, the diagnosis systems (e.g., diagnosis system 1 to diagnosis system N, 10 to 30 and/or standard diagnosis system 40) may be re-trained using the stored information.

For example, after a lapse of a predetermined time, the diagnosis system 2 may be re-trained using a re-training data set including the specific biometric data labeled as the consensus diagnosis result (e.g., O).

When the diagnosis system 2 is a diagnosis system customized for a specific diagnoser, the consent of the specific diagnoser may be required.

According to the technical idea of the present disclosure, it is possible to re-train each of the diagnosis systems (e.g., diagnosis system 1 to diagnosis system N, 10 to 30 and/or standard diagnosis system 40) by collecting biometric data that output diagnosis results different from the consensus diagnosis result. In other words, the biometric data stored by the consensus diagnosis system 100 and outputting a diagnosis result different from the consensus diagnosis result may be fed back to the individual diagnosis system as training data. In this case, there is an effect that the bias (e.g., according to the annotator's propensity or the bias of the training data itself) of each diagnosis system (e.g., diagnosis system 1 to diagnosis system N, 10 to 30 and/or standard diagnosis system 40) is improved.

The method for providing a customized diagnosis system according to an embodiment of the present disclosure may be implemented as computer-readable codes on a computer-readable recording medium. A computer-readable recording medium includes all types of recording devices in which data that may be read by a computer system is stored. Examples of a computer-readable recording medium include ROM, RAM, CD-ROM, magnetic tape, hard disk, floppy disk, optical data storage device, and the like. In addition, the computer-readable recording medium is distributed in computer systems connected through a network, so that computer-readable codes may be stored and executed in a distributed manner. In addition, functional programs, codes, and code segments for implementing the present disclosure may be easily inferred by programmers in the technical field to which the present disclosure belongs.

Although the present disclosure has been described with reference to an embodiment shown in the drawings, this is only exemplary, and those skilled in the art will understand that various modifications and equivalent other embodiments are possible therefrom. Therefore, the true technical protection scope of the present disclosure should be determined by the technical idea of the attached claims.

### Industrial Applicability

The present disclosure may be used for a method for providing a customized diagnosis system and a system thereof.

## Claims

1. A method for providing a customized diagnosis system, the method comprising:
transmitting, by a reference diagnosis system, M (M is an integer of 2 or more) pieces of unannotated training data for customization to a first diagnoser system;
receiving, by the reference diagnosis system, the M pieces of training data for customization annotated by a first diagnoser side from the first diagnoser system; and
reflecting the received M pieces of annotated training data for customization in the reference diagnosis system,
wherein the M pieces of training data for customization annotated by the first diagnoser side are reflected in a neural network for the reference diagnosis system under a condition that a predetermined price is paid.

2. The method of claim 1, comprising:
receiving diagnosis data from the first diagnoser system, generating a diagnosis result, and transmitting the diagnosis result to the first diagnoser system, by the reference diagnosis system in which the M pieces of annotated training data for customization are reflected.

3. The method of claim 1, wherein the reference diagnosis system sets the price to be larger as the M is larger or according to a ratio of the M to the number of training data learned by the reference diagnosis system.

4. The method of claim 1, wherein the reference diagnosis system is a system that comprises an individual diagnosis system set comprising a plurality of individual diagnosis systems and performs diagnosis through consensus based on individual diagnosis results output by the plurality of individual diagnosis systems.

5. The method of claim 4, wherein the reflecting of the received M pieces of annotated training data for customization in the reference diagnosis system comprises re-training at least some of the plurality of individual diagnosis systems using the M pieces of annotated training data for customization.

6. The method of claim 4, wherein the M pieces of training data for customization comprises data in which at least two of the individual diagnosis systems included in the individual diagnosis system set output different diagnosis results.

7. A method for providing a customized diagnosis system, comprising:
transmitting, by a reference diagnosis system, M (M is an integer of 2 or more) pieces of unannotated training data for customization to a first diagnoser system, the reference diagnosis system comprising an individual diagnosis system set comprising a plurality of individual diagnosis systems;
receiving, by the reference diagnosis system, the M pieces of training data for customization annotated by a first diagnoser side from the first diagnoser system;
reflecting the received M pieces of annotated training data for customization in any one first individual diagnosis system included in the reference diagnosis system set;
receiving a diagnosis request comprising diagnosis data to be diagnosed by the reference diagnosis system from a predetermined diagnosis request system; and
determining, according to the received diagnosis request, a type of diagnosis result corresponding to the diagnosis request as a first individual diagnosis result of the first individual diagnosis system, and transmitting a diagnosis result comprising the first individual diagnosis result output by the first individual diagnosis system receiving input data to the diagnosis request system.

8. A computer program stored in a non-transitory computer readable recording medium installed in a data processing device and for performing the method of any one of claims 1 to 7.

9. A data processing system, comprising:
a processor; and
a storage device in which a program is stored,
wherein the method of any one of claims 1 to 7 is performed by the program executed by the processor.

10. A customized diagnosis system, comprising:
a processor;
a storage device in which a program executed by the processor is stored,
wherein the storage device comprises at least one neural network, and
the program is configured to:
transmit M (M is an integer of 2 or more) pieces of unannotated training data for customization to a first diagnoser system, receive the M pieces of training data for customization annotated by a first diagnoser side from the first diagnoser system, and reflect the received M pieces of annotated training data for customization in a reference diagnosis system, and
wherein the M pieces of training data for customization annotated by the first diagnoser side are reflected in the neural network under a condition that a predetermined price is paid.

11. A customized diagnosis system, comprising:
a processor; and
a storage device in which a program executed by the processor is stored,
wherein the storage device comprises a neural network corresponding to a plurality of individual diagnosis systems, and
wherein the program is configured to:
transmit M (M is an integer of 2 or more) pieces of unannotated training data for customization to a first diagnoser system, and when receiving the M pieces of training data for customization annotated by a first diagnoser side from the first diagnoser system, reflect the received M pieces of annotated training data for customization to a first neural network corresponding to a first individual diagnosis system among the plurality of individual diagnosis systems, and
when receiving a diagnosis request comprising diagnosis data to be diagnosed from a predetermined diagnosis request system, determine, according to the received diagnosis request, a type of diagnosis result corresponding to the diagnosis request as a first individual diagnosis result of a first individual diagnosis system, and transmit a diagnosis result comprising the first individual diagnosis result output by the first individual diagnosis system receiving input data to the diagnosis request system.
